# EUROPEAN PATENT APPLICATION

(11) **EP 2 918 660 A1**
(43) Date of publication of application: **16.09.2015**
(21) Application number: 15158892.8
(22) Date of filing: 12.03.2015
(51) Int. Cl.: C10G 3/00, C07C 67/303, C07C 69/24, C07C 51/36, C07C 53/126, C11C 3/12

(54) **Process for low-hydrogen-consumption conversion of renewable feedstocks to alkanes**

(30) Priority: 14.03.2014 US 201414211333
(71) Applicant: Energy & Environmental Research Center Foundation, Grand Forks, North Dakota 58202-9017 (US)
(72) Inventor: Aulich, Ted R., Grand Forks, ND North Dakota 58201 (US); Wocken, Chad A, Grand Forks, ND North Dakota 58201 (US); Sharma, Ramesh K, Grand Forks, ND North Dakota 58201 (US)
(74) Representative: Walker, Ross Thomson

(57) **Abstract**

A process relating to the manufacture of hydrocarbons, particularly paraffins/alkanes, from fatty acid feedstocks. More specifically, a process relating to the manufacture of paraffins/alkanes from fatty acid feedstocks comprising an olefinic bond saturation followed by a deoxygenation process carried out using decarboxylation achieving a maximum feedstock conversion to a paraffin product while consuming a minimum amount of hydrogen.

## Description

### FIELD OF THE INVENTION

This process relates to the manufacture of hydrocarbons, particularly paraffins/alkanes, from fatty acid feedstocks. More specifically, the process relating to the manufacture of paraffins/alkanes from fatty acid feedstocks comprises an olefinic bond saturation followed by a deoxygenation step carried out using decarboxylation.

### BACKGROUND OF THE INVENTION

Concern for the environment and an increasing demand for petroleum-alternative fuels and chemicals are motivating producers to utilize renewable feedstocks. However, when applied to renewables processing, commercial refining processes originally developed for petroleum feedstocks often require significant adjustment to deal with the higher oxygen levels associated with most renewable feedstocks, which typically requires significant hydrogen consumption. Although vegetable oil-, animal fat-, and algae oil-derived fatty acids represent a potential petroleum replacement for fuel and chemical applications because of their long, straight, and mostly saturated hydrocarbon chains, neat fatty acid mixtures display inferior properties versus petroleum, such as high viscosity and chemical instability, that affect their direct use as fuels.

Fatty acids have been used as raw materials for manufacture of a wide range of products, including lubricants, polymers, fuels, solvents, and cosmetics. Fatty acids are generally obtained from wood pulping processes or by hydrolysis of triglycerides of vegetable or animal origin. Naturally occurring triglycerides are usually esters of glycerol and straight-chain, even-numbered carboxylic acids having 10-26 carbon atoms. Most common fatty acids contain 16, 18, 20, or 22 carbon atoms. Fatty acids may either be saturated or contain one or more unsaturated bonds. Unsaturated fatty acids are often olefinic, with *cis* configuration carbon-carbon double bonds. The unsaturated linkages occur in preferred positions in the carbon chain, the most common of which is the "omega 9" position as in oleic acid (C18:1) and erucic acid (C22:1). Polyunsaturated acids generally have a methylene-interrupted arrangement of *cis*-olefinic double bonds.

Saturated long straight-chain fatty acids (C10:0 and higher) are solid at room temperature, which makes their processing and use difficult in a number of applications. While unsaturated longer-chain fatty acids like oleic acid are easy-to-process liquids at room temperature, they are relatively unstable because of their double (olefinic) bond(s).

Conventional approaches for converting vegetable oils and/or fatty acid mixtures into fuels and chemicals comprise transesterification, hydrogenation, and cracking, among others. Triglycerides, which form the main component in vegetable oils, are converted into their corresponding esters by the transesterification reaction with an alcohol in the presence of catalysts. However, poor low-temperature properties of the products obtained limit their wider use as fuels in regions with colder climatic conditions, without additional processing such as filtration to remove materials that have higher-temperature gel points. Further, SAE International Paper No. 961086 (Schmidt, K.; Gerpen J.V.) teaches that the presence of oxygen in esters results in undesirable higher emissions of oxides of nitrogen (NOₓ) in comparison to conventional diesel fuels.

Thermal and catalytic cracking of biomaterials like vegetable oils and animal fats leads to a wide spectrum of products. U.S. Patent No. 5,233,109 describes an example of such a process using catalysts containing alumina and another component, such as silica or aluminosilicate. The reactions are generally unselective and result in formation of less valuable products. Further, the unsaturated and aromatic hydrocarbons present in the liquid fraction make these products unattractive for the diesel pool.

U.S. Patents Nos. 4,992,605 and 5,705,722 describe processes for the production of diesel fuel additives by reductive conversion of bio-oils into saturated hydrocarbons under hydroprocessing conditions. The reduction of a carboxylic group into a methyl group requires significant hydrogen partial pressure and results in significant hydrogen consumption. Additionally, the high hydrogen partial pressure requirement also effects the occurrence of undesirable side reactions such as methanation and the reverse water-gas shift reaction, which further increase hydrogen consumption. High hydrogen consumption limits the use of such processes, especially in refineries with limited excess hydrogen availability due to major hydrotreating requirements driven by the need to comply with environmental regulations or in stand-alone biorefineries without access to affordably priced hydrogen.

Undesired oxygen may be removed from fatty acids or esters by deoxygenation. The deoxygenation of bio-oils and fats to paraffinic hydrocarbons suitable as diesel fuel and/or as chemical intermediates may be performed in the presence of catalysts under hydroprocessing conditions. During hydrodeoxygenation conditions, oxygen is replaced with hydrogen, typically at a replacement ratio of 2 moles of hydrogen for 1 mole of oxygen. Therefore, this reaction requires rather high amounts of hydrogen while additional hydrogen is consumed in side reactions as well.

Decarboxylation-as opposed to deoxygenation-of fatty acids comprises removal of a CO₂ group and its replacement with a hydrogen atom and results in the yield of hydrocarbons with one carbon atom less than the original molecules from which they were derived. The feasibility of decarboxylation varies greatly with the type of carboxylic acid used as the starting material. Activated carboxylic acids containing electron-attracting substituents in the "alpha" or "beta" position with respect to the carboxylic group lose carbon dioxide spontaneously at slightly elevated temperatures. In this case, the RC-COOH bond is weakened by the electron shift along the carbon chain.

The majority of fatty acids are, however, not activated. The positive induction effect of the carbon chain evokes a high electron density in the position alpha to the carboxylic group, thus making the release of CO₂ difficult. Although the decarboxylation of activated and nonactivated carboxylic acids is thermodynamically comparable, the activation energy is significantly higher in the latter case. Therefore, relatively severe (high temperature/ pressure) conditions or the presence of a catalyst are required to overcome the activation energy barrier.

The fusion of alkaline salts of fatty acids with their corresponding hydroxides to yield hydrocarbons is known technology originally developed in the 19th century. The reaction is highly unselective and results in formation of ketones and cracking products at low conversion rates, as well as formation of undesired highly alkaline wastes. Further, a number of decarboxylation reactions have been developed and are used mainly in organic synthesis. Most of them proceed via free radical mechanisms. U.S. Patent No. 4,262,157 discloses a decarboxylation process utilizing diazacycloalkenes and Cu salts, wherein lauric acid reacts to form *n*-undecane (C11) with 51 % yield at 320°C. Decarboxylation of unsaturated acids to form hydrocarbons is also described. Indirect decarboxylation routes are also known, involving transformation of carboxylic acids into their corresponding halides, followed by their dehalogenation. The Hunsdiecker and Kochi reactions are examples of such reactions, and both proceed via free radical mechanisms.

Available alternative routes involve electrochemical and photocatalytic decompositions. An example of electrochemical decomposition is Kolbe electrolysis, wherein the reaction is started by anodic monoelectron oxidation leading to the formation of carboxylate radicals. Their subsequent decarboxylation results in probable formation of hydrocarbon radicals. Their dimerization-or less often, disproportionation-leads to termination of the free radical reaction. Electrolytic systems for hydrocarbon synthesis usually comprise aqueous solvents, organic cosolvents, added salts, and platinum electrodes. Under such conditions, the reaction yields 50%-90% coupling of hydrocarbon products. The main side products comprise 1-unsaturated hydrocarbons formed via disproportionation. A similar radical mechanism applies also for photocatalytically initiated reactions.

Two-step deoxygenation of oxygen-containing bio-oil compounds is described by Parmon et al., Catalysis Today 35 (1997) 153-162. The model compound, phenol, is, in a first process, treated with carbon monoxide over bimetallic alloy RhCu. The product, benzoic acid, consequently decarboxylates in the presence of PtPd or RuPd alloys in the second step.

The complexity of the decarboxylation reactions listed above and/or the low yield and often hazardous materials applied in the reactions are the main drawbacks of these approaches. Decarboxylation of carboxylic acids to hydrocarbons by contacting carboxylic acids with heterogeneous catalysts was suggested by Maier, W.F. et al., Chemische Berichte (1982), 115(2), 808-12. They tested Ni/Al₂O₃ and Pd/SiO₂ catalysts for decarboxylation of several carboxylic acids. During the reaction, the vapors of the reactant passed through a catalytic bed together with hydrogen. Hexane represented the main product of the decarboxylation of the tested compound heptanoic acid. When nitrogen was used instead of hydrogen, no decarboxylation was observed.

U.S. Patent No. 4,554,397 discloses a process for the manufacture of linear olefins from saturated fatty acids or esters. The catalytic system consists of nickel and at least one metal selected from the group consisting of lead, tin, and germanium. According to the examples, when other catalysts, such as Pd/C, were used, low catalytic activity, cracking to saturated hydrocarbons, or formation of ketones (when Raney-Ni was used) was observed.

Decarboxylation, accompanied with hydrogenation of oxo-compound, is described in Laurent, E., Delmon, B., Applied Catalysis, A: General (1994), 109(1), 77-96 and 97-115, wherein hydrodeoxygenation of biomass-derived pyrolysis oils over sulfided CoMo/*gamma*-Al₂O₃ and NiMo/*gamma*-Al₂O₃ catalysts was studied. Diethyldecanedioate was used among others as a model compound, and it was observed that the rates of formation of the decarboxylation product (nonane) and the hydrogenation product (decane) were comparable under hydrotreating conditions (260°-300°C, 7 MPa, in hydrogen). NiMo/*gamma*-Al₂O₃ showed slightly higher selectivity toward decarboxylation products in comparison to CoMo/*gamma-*Al₂O₃ catalyst. The presence of hydrogen sulfide, in contrast to ammonia, also promoted the decarboxylation, particularly when NiMo catalysts were used.

A process for converting an ester-containing vegetable oil into hydrocarbons is disclosed in GB 1,524,781. The conversion to hydrocarbons is performed over a catalyst containing an admixture of silica-alumina with an oxide of a transition state metal of groups IIA, IIIA, IVA, VA, VIA, VIIA, or VIIIA of the periodic table at the reaction temperatures of 300°-700°C. The products formed are reported to be free from oxygenated compounds (other than carbon dioxide and water). According to the examples cited, extensive cracking was observed.

U.S. Patents 7,491,858, 7,816,570, 8,039,682, and 8,247,632 and Patent Application No. 20120029250 describe fatty acid catalytic hydrodeoxygenation processes that include a decarboxylation component, but the described processes also include hydrogen-consuming decarbonylation and/or reduction reactions as a means of achieving deoxygenation, and some of the processes include cyclization and/or aromatization reactions (resulting in fatty acid conversion to naphthenes and aromatics) as a means of achieving decreased hydrogen consumption.

As the above demonstrates, a need exists for an economically viable catalytic method for the quantitative conversion of fatty acid resources to paraffinic hydrocarbons through the use of selective decarboxylation-as opposed to decarbonylation and/or deoxygenation-as a means of achieving improved economics through reduced hydrogen consumption.

### SUMMARY OF THE INVENTION

This process relates to the manufacture of hydrocarbons, particularly paraffins or alkanes, from fatty acid feedstocks. More specifically, the process relates to the manufacture of paraffins/alkanes, from fatty acid feedstocks comprising an olefinic bond saturation followed by a deoxygenation process carried out using decarboxylation, achieving a maximum feedstock conversion to a paraffin product while consuming a minimum amount of hydrogen.

### BRIEF DESCRIPTION OF THE DRAWINGS

In the drawings:
FIG. 1 illustrates a comparison of reduction, decarbonylation, and decarboxylation based on hydrogen consumption.
FIG. 2 illustrates the olefinic bond saturation process and decarboxylation process.
FIG. 3 illustrates the gas chromatogram of soy fatty acid-derived alkanes produced via the Strege one-step process of simultaneous olefinic bond saturation-deoxygenation.

### DETAILED DESCRIPTION OF THE INVENTION

### Terms and Definitions

The following explanations of terms and abbreviations are provided to better describe the present disclosure and to guide those of ordinary skill in the art in the practice of the present disclosure. As used herein, "comprising" means "including," and the singular forms "a" or "an" or "the" include plural references unless the context clearly dictates otherwise. The term "or" refers to a single element of stated alternative elements or a combination of two or more elements, unless the context clearly indicates otherwise.

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, suitable methods and materials are described below. The materials, methods, and examples are illustrative only and not intended to be limiting. Other features of the disclosure are apparent from the following detailed description and the claims.

Unless otherwise indicated, all numbers expressing quantities of components, percentages, temperatures, times, and so forth, as used in the specification or claims are to be understood as being modified by the term "about." Accordingly, unless otherwise indicated, implicitly or explicitly, the numerical parameters set forth are approximations that may depend on the desired properties sought and/or limits of detection under standard test conditions/methods. When directly and explicitly distinguishing embodiments from discussed prior art, the embodiment numbers are not approximates unless the word "about" is recited.

Definitions of particular terms, not otherwise defined herein, may be found in Richard J. Lewis, Sr. (ed.), Hawley's Condensed Chemical Dictionary, published by John Wiley & Sons, Inc., 1997 (ISBN 0-471-29205-2). In order to facilitate review of the various embodiments of the disclosure, the following explanations of specific terms are provided.

As used herein, "catalyst" refers to a substance, usually present in small amounts relative to reactants, that increases the rate of a chemical reaction without itself being consumed or undergoing a chemical change. A catalyst also may enable a reaction to proceed under different conditions (e.g., at a lower temperature) than otherwise possible. Catalysts typically are highly specific with respect to the reactions in which they participate. Some catalysts have a limited lifetime, after which they must be replaced or regenerated. For example, reaction products or by-products may deposit on the surface or within the pores of a catalyst, reducing its activity.

As used herein, "cracking" refers to a refining process involving decomposition and molecular recombination of long-chain hydrocarbons into shorter hydrocarbons. Catalytic cracking occurs when heated hydrocarbons are passed over metal oxide and/or metallic catalysts (e.g., silica-alumina or platinum). In hydrocracking, a catalyst is used, and hydrogen is added to produce primarily saturated hydrocarbons. Hydrocracking can also produce unsaturated and aromatic hydrocarbons.

As used herein, "decarboxylation" refers to a chemical reaction in which carbon dioxide is removed from a chemical compound. For example, a fatty acid may be decarboxylated to produce a hydrocarbon and carbon dioxide: RCOOH → RH + CO₂.

As used herein, "fatty acid" refers to a carboxylic acid having a long, unbranched, aliphatic chain or tail. Naturally occurring fatty acids commonly contain from 4 to 28 carbon atoms (usually an even number) including the carbon atom in the carboxyl group. Free fatty acids can be represented by the general formula RCOOH, where R is a saturated (i.e., all single bonds) or unsaturated (i.e., contains one or more double or triple bonds) aliphatic chain. Saturated fatty acids have only single bonds in the carbon chain and can be described by the general formula CH₃(CH₂)ₓCOOH. Unsaturated fatty acids have one or more double or triple bonds in the carbon chain. Most natural fatty acids have an aliphatic chain that has at least eight carbon atoms and an even number of carbon atoms (including the carbon atom in the carboxyl group). The fatty acid may be a liquid, semisolid, or solid. As used herein, the term "fatty acids" refers to a mixture of fatty acids of varying carbon number and degree of saturation.

As used herein, "olefin" refers to an unsaturated aliphatic hydrocarbon having one or more double bonds. Olefins with one double bond are alkenes; olefins with two double bonds are alkadienes or diolefins. Olefins typically are obtained by cracking petroleum fractions at high temperatures.

As used herein, "Weight hourly space velocity (WHSV)" refers to the weight of feed flowing per weight of catalyst per hour.

### Process

Virtually all fatty acid feedstocks comprise mixtures of saturated and unsaturated species, while some such feedstocks can comprise up to 100% unsaturated species. Because unsaturated linkages (olefinic bonds) between carbon molecules (often referred to as "double bonds") are associated with high electron density, these sites are susceptible to cracking and other undesirable hydrogen-consuming reactions under the relatively high severity catalytic reaction conditions typically utilized to effect fatty acid deoxygenation via decarboxylation, decarbonylation, reduction, or any combination of these. To minimize such undesirable reactions, a low-severity catalytic reaction to effect double bond saturation is used to convert all double bonds to single bonds prior to subjecting the fatty acids to higher-severity decarboxylation, thereby eliminating high electron density sites and stabilizing the fatty acids against the occurrence of hydrogen-consuming cracking reactions. Although the low-severity catalytic reaction to effect double bond saturation consumes some hydrogen, hydrogen consumption is necessary to saturate olefin bonds and achieve the desired overall process output of a 100% paraffin product, which means a certain minimum level of hydrogen consumption is unavoidable. What is desired to avoid is hydrogen waste during the process.

Because olefinic bond saturation is a very low severity process, when properly executed it results in no or, at the worst, minimal cracking and the production of methane and other hydrogen-containing/consuming gases during the catalytic reaction. Such a process means that no hydrogen is wasted on "capping" free radicals and, because the only gaseous product emerging from the reactor vessel during the low-severity catalytic reaction to effect double bond saturation is hydrogen, little or no expensive hydrogen purification is needed prior to recycle of 100% of the unconsumed hydrogen, which also translates to no wasted hydrogen.

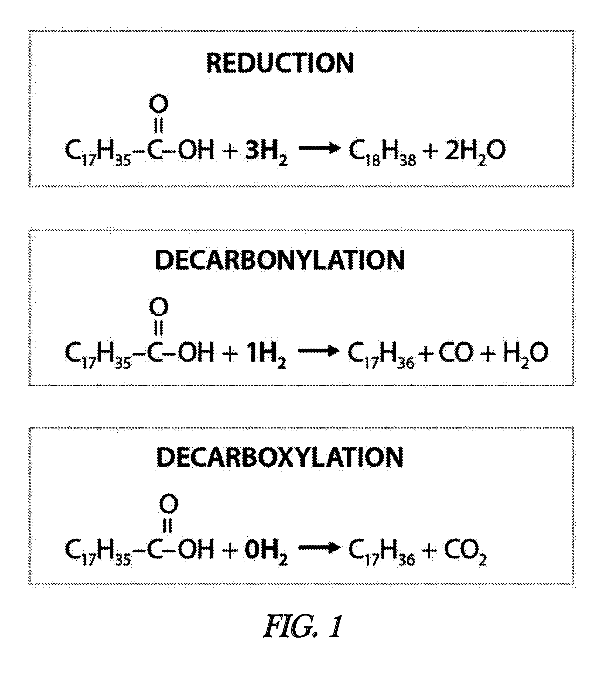

Following the low-severity catalytic reaction to effect double bond saturation, saturated fatty acids undergo a highly selective catalytic decarboxylation process. A reason for focusing exclusively on decarboxylation rather than decarbonylation or reduction is that decarboxylation results in the lowest hydrogen consumption of these three fatty acid deoxygenation routes, as illustrated in drawing FIG. 1.

Such a selective catalytic decarboxylation process comprises a two-step process for selective decarboxylation of fatty acids to effect their conversion to paraffins, with decarboxylation including oxygen removal in the form of CO₂. Under suitable conditions of the selective catalytic decarboxylation process, hydrogen is required only for initial catalyst reduction, catalyst maintenance, and saturation of olefinic bonds. Consequently, hydrogen consumption is reduced to a minimum.

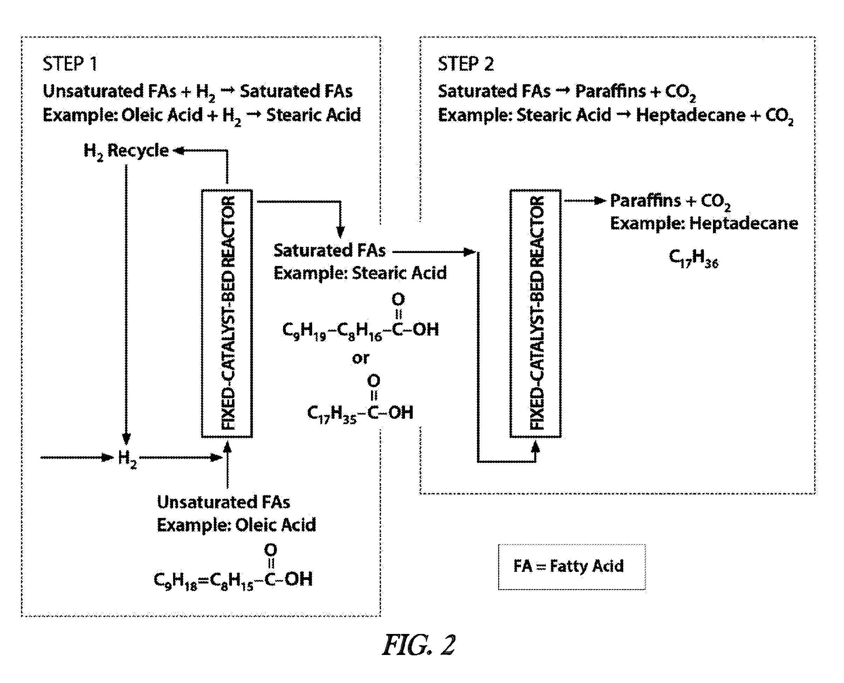

In such selective catalytic decarboxylation processes, the low-severity catalytic reaction to effect double bond saturation comprises bringing an appropriate fatty acid-based feedstock containing species with unsaturated/olefinic bond linkages into contact with an optionally pretreated heterogeneous catalyst selected from supported catalysts containing one or more Group VIII or VIA metals for carrying out an olefinic bond saturation reaction at a temperature in the range of approximately 50°-250°C, at a hydrogen pressure in the range of approximately 0.1-30 MPa (with hydrogen in its pure form or mixed at low levels [about 5 vol%] with an inert carrier gas comprising nitrogen, helium, argon, or any combination of these) to yield a product mixture of saturated fatty acid-based species. The heterogeneous catalyst is optionally pretreated with hydrogen at a temperature in the range of approximately 100°-500°C. Pretreatment of the heterogeneous catalyst is preferable as it ensures the activity of the catalyst. The olefinic bond saturation reaction is carried out in liquid phase; thus the reaction pressure is higher than the saturation vapor pressure of the feedstock at a given reaction temperature. The reaction pressure ranges from approximately atmospheric pressure to approximately 30 MPa, based on the properties of the feedstock. Excess hydrogen (hydrogen that is unconsumed by the olefinic bond saturation reaction) is recycled, as shown in drawing FIG. 2. Because of the low severity of the saturation reaction and because the low-severity conditions minimize or eliminate the occurrence of cracking reactions that cleave single reactant molecules into two or more smaller molecules, including gaseous species, hydrogen is the only gaseous specie present in the low-severity catalytic reaction to effect double bond saturation of gas. This means that the recycling of 100% of unreacted/unconsumed hydrogen can be accomplished without the need for hydrogen cleanup/purification, which is expensive and typically results in some level of hydrogen waste. In addition to making hydrogen recycling easier and less expensive than processes in which hydrogen cleanup/purification is required, the nonoccurrence of cracking reactions also eliminates the need for hydrogen to effect free radical capping reactions, thereby further minimizing hydrogen consumption.

In accordance with the selective catalytic decarboxylation process, the process includes bringing the saturated fatty acid-based product of the low-severity catalytic double bond saturation reaction into contact with an optionally pretreated heterogeneous catalyst selected from supported catalysts containing one or more Group VIII or VIA metals, using a selective decarboxylation reaction carried out at a temperature in the range of approximately 200°-450°C, under a pressure ranging from approximately atmospheric (0.1 MPa) to approximately 150 MPa, to yield a product mixture of paraffins/alkanes with each individual paraffin specie having one less carbon atom than the fatty acid specie from which it was derived. The heterogeneous catalyst is optionally pretreated with hydrogen at a temperature in the range of approximately 100°-500°C. Pretreatment of the heterogeneous catalyst is preferable as it helps ensure the activity of the catalyst. The decarboxylation reaction is carried out in liquid phase; thus the reaction pressure is higher than the saturation vapor pressure of the feedstock at a given reaction temperature. The reaction pressure ranges from approximately atmospheric pressure to approximately 150 MPa, based on the properties of the feedstock. A gas flow comprising an inert gas such as nitrogen, helium, argon, other, or any combination thereof may be used for removing gaseous products formed during the reaction. In some cases, low-level hydrogen addition to the gas flow may be needed to maintain catalyst activity and decarboxylation performance. In some cases, a solvent may be added to the saturated fatty acid-based species reactant mixture, with the solvent comprising one or more of a combination of paraffin(s), isoparaffin(s), naphthene(s), aromatic(s), and recycled decarboxylation reaction alkane product mixture.

In various embodiments, the ratio of moles of the paraffin product generated by decarboxylation reactions and decarbonylation reactions to the moles of the paraffin product generated by reduction reactions and deoxygenation reactions can be any suitable ratio, such as about 0.1:1 to about 10:1, about 0.3:1 to about 3.2:1, about 0.6:1 to about 1.6:1, or about 0.1:1 or less, or about 0.2:1, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.2, 2.4, 2.6, 2.8, 3, 3.5, 4, 4.5, 5, 5.5, 6, 7, 8, 9, or about 10:1 or more.

In various embodiments, the weight percent conversion of at least one of the unsaturated fatty acids and the unsaturated fatty acid esters to the paraffin product can be any suitable wt%, such as about 10 wt% to about 100 wt%, about 20 wt% to about 90 wt%, about 25 wt% to about 80 wt%, or about 10 wt% or less, or about 15 wt%, 20, 25, 30, 35, 40, 45, 50, 55, 60, 65, 70, 75, 80, 85, 90, 95, 96, 97, 98, 99, 99.9, or about 99.99 wt% or more.

In various embodiments, the olefin bond saturation process and the deoxygenation process together consume any suitable mass of hydrogen per 100 grams of paraffin product produced, such as about 0.1 g to about 5 g, about 0.5 g to about 2.5 g, about 1 g to about 2 g, about 0.1 g or less, or about 0.2 g, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.2, 2.4, 2.6, 2.8, 3, 3.5, 4, 4.5, or about 5 g or more.

### Feedstock

The fatty acid feedstock comprises renewable sources, such as fats and oils from plants and/or animals and/or fish and compounds derived from them. Examples of suitable feedstocks are plant and vegetable oils and fats, animal fats and oils, fish fats and oils, and mixtures thereof containing fatty acids and/or fatty acid esters. Particularly suitable materials are fatty acid-based mixtures derived from wood-based and other plant-based and vegetable-based fats and oils such as soybean oil; corn oil; rapeseed/canola oil; tall oil; sunflower oil; hempseed oil; olive oil; linseed oil; mustard oil; palm oil; peanut oil; castor oil; coconut oil; oil from algae; fats contained in plants bred by means of genetic manipulation; animal-based fats such as lard, tallow, train oil, and fats contained in milk; recycled fats from the food industry; and mixtures of the above.

Preferably, the feedstock comprises C8-C24 fatty acids, derivatives of said fatty acids, such as esters of said fatty acids as well as triglycerides of said fatty acids, metal salts of said fatty acids, or combinations thereof. The fatty acids or fatty acid derivatives, such as esters, may be produced via hydrolysis of bio-oils or by their fractionation or by esterification reactions of triglycerides. Suitable triglyceride fractions of rapeseed oil, linseed oil, sunflower oil, tallow and lard, and fractions of tall oil are used as the feedstock.

The paraffin/alkane products obtained utilizing the method according to the invention have one carbon atom less than the original fatty acid or fatty acid-based specie from which each product paraffin specie was derived.

### Reaction Conditions

The olefinic bond saturation process and the decarboxylation reaction conditions may vary depending on the properties of the feedstock used. Both the olefinic bond saturation process and the decarboxylation reaction are carried out in liquid phase. The olefinic bond saturation reaction is carried out at a temperature in a range of approximately 50°-250°C under a hydrogen pressure in the range of approximately 0.1-30 MPa. Hydrogen is required for olefinic bond saturation, and a pressure higher than the saturation vapor pressure of the feedstock is required to maintain the reactants in the liquid phase. The decarboxylation reaction is carried out at a temperature in the range of approximately 200°-450°C, under an inert gas or inert gas-low level hydrogen mixture pressure ranging from approximately atmospheric (0.1 MPa) to approximately 150 MPa.

### Solvent

An optional solvent is selected from the group comprising paraffins, isoparaffins, naphthenes, and aromatic hydrocarbons in the boiling range of approximately 150°-350°C, recycled decarboxylation reaction product, and mixtures thereof; preferably, recycled decarboxylation reaction product is used as the solvent.

### Gas Flow

In the olefinic bond saturation process, hydrogen or a hydrogen-inert gas mixture is used as a carrier gas and to provide hydrogen for olefinic bond saturation, and in the decarboxylation reaction, an inert gas such as nitrogen, helium, or argon, or an inert gas mixed with hydrogen (for catalyst activity and performance maintenance), or any combinations thereof may be used for removing gaseous products formed during the reaction. The gas flow may be combined with the feedstock or fed to the reaction mixture. Hydrogen concentration in the olefinic bond saturation process carrier gas may vary in the range of from approximately 5-100 vol% and in the decarboxylation reaction carrier gas from approximately 1-15 vol%.

### Catalyst

The catalyst in both the olefinic bond saturation and the decarboxylation reactions is a supported heterogeneous catalyst comprising at least one active elemental metal selected from the metals belonging to Group VIII and/or Group VI of the periodic table. The same catalyst or two different catalysts may be used for the olefinic bond saturation reaction and the decarboxylation reaction. Suitable metals used as the catalyst comprise Pt, Pd, Ni, NiMo, CoMo, Ir, Ru, Rh, and any combination thereof. A preferable metal used as the catalyst comprises Pd, supported on oxides, mesoporous materials or carbonaceous supports, such as Al₂O₃, SiO₃, Cr₂O₃, MgO, TiO₂, or C in the form of activated carbon or other structured carbon catalyst support, such as carbon fibers, carbon nanotubes attached to monoliths, and carbon cloths. Loading of the active metal varies in the range of approximately 0.5-20 wt%. When nickel makes up the catalyst, the loading varies in the range of approximately 2-55 wt%.

Either or both reaction(s), the olefinic bond saturation reaction and the decarboxylation reaction, may be carried out in batch, semibatch, or continuous mode in reactors such as trickle-bed, continuous tubular, or continuous stirred-tank reactors for separation of the gaseous CO₂ and the paraffin/alkane product.

Two advantages of the olefinic bond saturation reaction and the decarboxylation reaction derive from their ability to effect conversion of fatty acid-based feedstocks to paraffins with minimum hydrogen consumption and with minimum occurrence of cracking reactions. These advantages translate to higher energy efficiency and lower capital and operating costs, yielding a product slate with maximum concentration of desired long-chain paraffins and minimum concentration of cracking-derived shorter-chain gaseous and low-volatility liquid paraffins.

Further, in the olefinic bond saturation process and the decarboxylation reaction, the oxygenated feedstock, such as C₈-C₂₄ fatty acids, as well as derivatives of said fatty acids, such as esters of said fatty acids, triglycerides of said fatty acids, or metal salts of said fatty acids, can be converted-with high selectivity-to desired paraffins/alkanes. Each individual paraffin product constituent has one less carbon atom than the fatty acid material from which it was derived, and the structure of each obtained paraffin product specie corresponds to the structure of the fatty acid material from which it was derived.

Conducting the reaction in a liquid phase is preferential and advantageous versus a gas-phase reaction. A gas-phase reaction requires high reaction temperature in order to vaporize feedstock, which causes decomposition of high-boiling feedstock compounds and supports endothermic side reactions as well as catalyst deactivation because of sintering and fouling. Maintaining the reactants in liquid phase also enables simpler and less expensive process control.

The olefinic bond saturation process and the decarboxylation reaction are illustrated in the following example. It is evident to a person skilled in the art that the scope of the olefinic bond saturation process and the decarboxylation reaction is not meant to be limited to this example.

### EXAMPLES

### Example 1

In the presented example of the invention, the conceptualized performance of the two-step olefinic bond saturation-decarboxylation process is compared to the performance of a patented one-step process (Strege, J. et al.; U.S. Patent 8,247,632) on the basis of overall hydrogen consumption in the conversion of a fatty acid mixture to a paraffin mixture. Table 1 illustrates the composition of a feedstock fatty acid mixture derived by steam hydrolysis of a soybean oil. Conducting the subject invention two-step olefinic bond saturation-decarboxylation reaction process under the conditions summarized in Table 2 will yield an alkane product with the approximate composition described in Table 3. Based on the quantity of hydrogen needed to effect saturation of the olefinic bonds present in the feedstock and assuming the occurrence of decarboxylation rather than decarbonylation and/or reduction as the principal means of effecting feedstock deoxygenation, approximately 1.3 grams of hydrogen is consumed in yielding 100 grams of the product described in Table 3.

In conducting the Strege process, the reactor system used comprised a tubular reactor with internal dimensions of 1.5 inches in diameter and 56 inches in length. Reactor heating to appropriate operating temperature was accomplished by means of heating elements affixed to the outside of the reactor tube. Liquid was supplied to the reactor by means of a high-pressure pump that drew fatty acid in the liquid state from a heated reservoir. The fatty acid was passed through a tubular preheater prior to introduction to the tubular reactor. Hydrogen was supplied from high-pressure cylinders, with the flow rate controlled by means of a mass flow controller. The pressure of the reactor system was controlled by means of a back-pressure controller located at the end of the reactor system. The end of the reactor system possessed a chiller and a pressure letdown system to aid in sample collection. Temperatures, pressures, and flow rates were controlled via computer-driven process control software.

**Table 1 - Composition of Soybean Oil-Derived**

| Fatty Acid Feedstock | |
|---|---|
| Fatty Acid | Composition, wt% |
| C16:0 | 10.4 |
| C16:1 | - |
| C18:0 | 3.6 |
| C18:1 | 25.3 |
| C18:2 | 54.8 |
| C18:3 | 5.1 |
| C20:0 | 0.7 |
| C20:1 | - |
| Others | 0.1 |

**Table 2 - Two-Step Fatty Acid-to-Alkanes Reaction Conditions**

| Reaction Condition | Step 1 - Olefinic Bond Saturation | Step 2 - Decarboxylation |
|---|---|---|
| Mode | Continuous | Continuous |
| Reactor Configuration | Fixed catalyst bed | Fixed catalyst bed |
| Catalyst | Group VIII metal(s) on support | Group VIII metal(s) on support |
| Carrier Gas | Hydrogen | Hydrogen ∼2% in 98% inert |
| Temperature | ∼150°C | ∼300°-450°C |
| Pressure | ∼15 MPa | Up to 150 MPa |

**Table 3 - Soy Fatty Acid Conversion to Alkanes via Two-Step**

| Decarboxylation Process - Approximate Product Slate | |
|---|---|
| Alkane | Composition, wt% |
| C15 | 10.4 |
| C17 | 88.8 |
| C19 | 0.7 |
| Other | 0.1 |

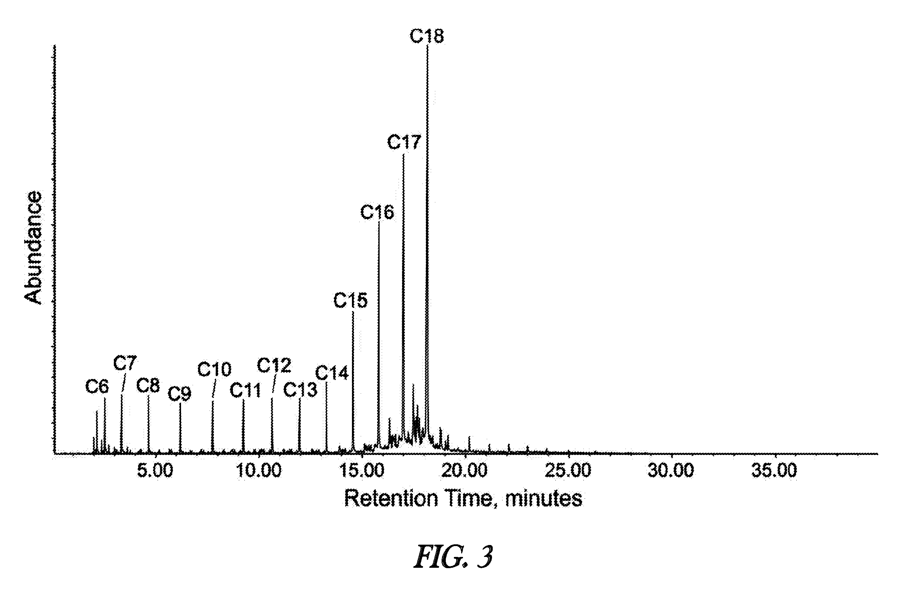

The reactor was charged with about 1.5 kilograms of a commercial hydrotreating catalyst. The catalyst bed was slowly warmed to the desired operating temperature while a steady flow of hydrogen was passed over the catalyst bed. A hydrogen flow of 50 standard cubic feet per hour (scfh), a liquid flow of 2 liters per hour (lph) of fatty acid, and a reactor pressure of 735 pounds per square inch (psi) were established. The temperature of the reactor was stabilized at 430°C. The fatty acid mixture described by Table 1 was pumped through the reactor and converted to the alkane mixture illustrated and described by FIG. 3 and Table 4, respectively. The recovered mass yield of liquid products was 95.8%. Analysis indicated that ∼85.0% of the mass of fatty acid had been converted to hydrocarbon and ∼10.8% converted to water, with the balance being converted to gaseous products.

As shown in Table 4 and FIG. 3, the one-step Strege process effected simultaneous fatty acid olefinic bond saturation, cracking (as evidenced by the presence of C4-C14 alkanes), and deoxygenation, with deoxygenation occurring via decarboxylation as well as decarbonylation and reduction (as evidenced by water formation). Resulting from hydrogen consumption because of decarbonylation, reduction, and cracking (wherein hydrogen is consumed by free radical capping) in addition to the required olefinic bond saturation, the one-step fatty acid-to-alkanes process requires approximately 3 grams of hydrogen per 100 grams of alkanes product or over twice as much hydrogen as required for the two-step process (1.3 grams hydrogen per 100 grams alkanes) described by the subject invention. Because of the significant capital and operating costs of either hydrogen transportation and storage or its production on-site, a 50% reduction in hydrogen input requirement represents a significant commercial advantage.

**Table 4. One-Step Fatty Acid Deoxygenation Product Hydrocarbon Distribution**

| Carbon Number | % *n*-Paraffin | % *iso*-Paraffin | % Cycloparaffin | % Olefin |
|---|---|---|---|---|
| 18 | 35.75 | 8.2 | - | 1.89 |
| 17 | 10.34 | 2.4 | - | 1.04 |
| 16 | 14.28 | 0.83 | - | - |
| 15 | 3.95 | 0.36 | - | - |
| 14 | 1.56 | 0.14 | 0.02 | - |
| 13 | 1.23 | 0.22 | - | - |
| 12 | 1.26 | 0.26 | - | 0.08 |
| 11 | 1.18 | 0.26 | - | - |
| 10 | 1.20 | 0.24 | 0.02 | 0.08 |
| 9 | 1.05 | 0.23 | 0.04 | - |
| 8 | 1.16 | 0.31 | 0.07 | - |
| 7 | 1.15 | 1.04 | 0.18 | - |
| 6 | 0.97 | 0.24 | 0.27 | 0.06 |
| 5 | 0.67 | 0.09 | 0.15 | - |
| 4 | 0.31 | - | - | - |
| 3 | 0.07 | - | - | - |
| Totals | 76.13 | 14.82 | 0.75 | 3.15 |

### Example 2

In this presented example of the invention, the utility and advantage of the two-step (olefinic bond saturation followed by decarboxylation) process is demonstrated by comparing the outputs of a catalytic decarboxylation process when operated with two different feedstocks: oleic acid and stearic acid. As shown below, both stearic and oleic acid are 18-carbon linear carboxylic acids with the only difference between the two acids being that oleic acid contains one olefinic (unsaturated) bond, while stearic acid contains no olefinic bonds.

Oleic acid: C₉H₁₈=C₈H₁₅-COOH

Stearic acid: C₁₇H₃₅-COOH

When oleic acid is subjected to a mild catalytic hydrogenation/saturation process, it is converted to stearic acid at a high yield, with essentially no cracking of oleic acid to smaller carboxylic acids. This is illustrated in Table 5, which compares the analyzed composition of a beef tallow fatty acid mixture to the analyzed composition of the beef tallow fatty acid mixture after undergoing mild hydrogenation via a commercially practiced industrial process. Although variability in the analytical method employed does not enable complete material balance closure, the values in the table show that conversion of all olefin bond-containing (unsaturated) C18 species to stearic acid is near 100% and that cracking of the unsaturated species has not occurred.

**Table 5 - Conversion of Oleic Acid to Stearic Acid via Commercial Hydrogenation Process**

| Carbon Chain Length: Number Olefin Bonds | Fatty Acid Name | Beef Tallow Fatty Acid Mixture | Hydrogenated Beef Tallow Fatty Acid Mixture |
|---|---|---|---|
| | | Fatty Acid, weight% | Fatty Acid, weight% |
| C8:0 | Caprylic | 1.1 | Not Detected |
| C10:0 | Capric | 0.4 | Not Detected |
| C12:0 | Lauric | 1.4 | 0.1 |
| C14:0 | Myristic | 3.2 | 2.9 |
| C16:0 | Palmitic | 23.4 | 26.2 |
| C16:1 | Palmitoleic | 1.0 | 0.6 |
| C17:0 | Margaric | 1.4 | 1.6 |
| C18:0 | Stearic | 19.2 | 65.6 |
| C18:1 | Oleic | 38.9 | 0.3 |
| C18:2 | Linoleic | 2.8 | Not Detected |
| C18:3 | Linolenic | 0.4 | Not Detected |
| | | | |
| Total | | 93.2 | 97.3 |

It is envisioned that the present invention could utilize-to effect Step 1 olefin bond saturation-the above-referenced hydrogenation process or one of several other commercially practiced hydrogenation processes. Following Step 1 conversion of unsaturated species to saturated species, Step 2 comprises catalytic decarboxylation with the objective of achieving maximum hydrocarbon (ideally, paraffin) yield with minimum hydrogen consumption. The decreased hydrogen requirement and yield improvement of the two-step process (present invention) are illustrated by comparing the outputs of the following tests performed with oleic acid and stearic acid feedstocks. While the stearic acid test represents Step 2 (decarboxylation of Step 1-saturated material) of the present invention, the oleic acid test represents a conventional one-step process encompassing olefin bond saturation and decarboxylation.

The tests were conducted using a 0.8-inch inside diameter by 5-inch-long tubular reactor with a fixed bed containing a commercial hydrotreating catalyst. The reactor was heated to appropriate operating temperature by placing it inside a heated fluidized bed of sand. Liquid feed was supplied to the reactor by means of a high-pressure pump that drew fatty acid in the liquid state from a heated reservoir. Hydrogen was supplied from high-pressure cylinders, with the flow rate controlled by means of a mass flow controller. The catalyst used was provided by product exiting the reactor system flowed through a 2-phase separator where liquids were collected by opening a manual valve. Gas exited the separator vessel and passed through an actuated control valve which provided pressure control for the system. Temperatures, pressures, and flow rates were controlled via computer-driven process control software. Table 6 summarizes the operating conditions utilized for each test. As shown, operating conditions were the same for both tests, with the exception of hydrogen flow (provided at the recommendation of the catalyst supplier to ensure maintenance of maximum catalyst activity). Additional hydrogen was supplied to the oleic acid test to ensure availability of sufficient hydrogen for olefin bond saturation.

**Table 6 - Summary of Operating Conditions**

| Reaction Condition | Stearic Acid | Oleic Acid |
|---|---|---|
| Mode | Continuous | Continuous |
| Reactor Configuration | Fixed Catalyst Bed | Fixed Catalyst Bed |
| Catalyst | Ni/Mo on Support | Ni/Mo on Support |
| Carrier Gas | 25% Hydrogen/75% Nitrogen | 25% Hydrogen/75% Nitrogen |
| Gas Feed Rate | 240 sccm¹ | 570 sccm |
| Temperature | 300°C | 300°C |
| Pressure | 600 psig² | 600 psig |
| Liquid Feed Rate | 2.5 mL/min³ | 2.5 mL/min |

| | | |
|---|---|---|
| ¹ Standard cubic centimeters per minute. ² Pounds per square inch (gauge). ³ Milliliters per minute. | | |

Liquid products from each test were collected and analyzed via gas chromatography-mass spectrometry. Results of the analyses are presented in Table 7. In Table 7, "conversion" refers to the percentage of input acid converted to nonacid hydrocarbon (primarily paraffin and olefin) products. Also in Table 7, "C17/C18 product ratio" refers to the mass ratio of 17-carbon paraffin product to 18-carbon paraffin product, which indicates the extent of occurrence of more desirable (less hydrogen-consuming) decarboxylation/decarbonylation versus less desirable (more hydrogen-consuming) reduction/deoxygenation.

**Table 7 - Stearic versus Oleic Acid - Conversion and Product Ratio**

| Test | Conversion, weight% | C17/C18 Product Ratio |
|---|---|---|
| Stearic Acid | 69 | 1.6 |
| Oleic Acid | 25 | 0.6 |

With virtually all chemical reactions, achieving theoretical performance at commercial scales is difficult, and it is understood that the subject invention is unlikely to achieve either 100% decarboxylation selectivity or 0% cracking. However, because both the subject invention and other fatty acid-to-paraffin processes require equal hydrogen consumption for olefinic bond saturation (about 1.3 grams per 100 grams product) and because the reduction reactions are the principal hydrogen consumers of the Strege process and other similar deoxygenation processes (equating to about 85% of the additional hydrogen consumption needed versus the subject invention), the ability to limit the occurrence of reduction reactions via the subject invention will translate to significantly decreased hydrogen consumption and concomitantly decreased processing cost.

While the invention has been described and illustrated in detail, it will be understood that the invention may be embodied otherwise without departing therefrom.

### Additional Embodiments.

The following exemplary embodiments are provided, the numbering of which is not to be construed as designating levels of importance:

Embodiment 1 provides a process for the manufacture of saturated hydrocarbons, the process comprising:
performing a olefinic bond saturation process on a feedstock comprising at least one of unsaturated fatty acids and unsaturated fatty acid esters, optionally comprising at least one of saturated fatty acids, saturated fatty acid esters, and triacylglycerides; and
performing a deoxygenation process on the feedstock including a decarboxylation process to yield a mixture of paraffins.

Embodiment 2 provides the process according to Embodiment 1, wherein the feedstock comprises at least about 20% by weight of unsaturated fatty acids or fatty acid alkyl esters.

Embodiment 3 provides the process according to any one of Embodiments 1-2, wherein the feedstock comprises about 50% to about 100% by weight of unsaturated fatty acids or fatty acid alkyl esters.

Embodiment 4 provides the process according to any one of Embodiments 1-3, wherein the fatty acids or fatty acid alkyl esters used as the feedstock have carbon numbers ranging from 8 to 26.

Embodiment 5 provides the process according to any one of Embodiments 1-4, wherein the feedstock comprises biological materials.

Embodiment 6 provides the process according to any one of Embodiments 1-5, wherein the olefinic bond saturation is carried out in the presence of a supported hydrogenation catalyst comprising one or more Group VIII metals of the periodic table and Group VIA metals of the periodic table, at a temperature of about 50°C to about 250°C at a pressure using hydrogen at a pressure of about 0.1 MPato about 30 MPa.

Embodiment 7 provides the process according to Embodiment 6, wherein the catalyst for olefinic bond saturation comprises at least one of Ni, Mo, Pd, and Co.

Embodiment 8 provides the process according to any one of Embodiments 6-7, wherein the olefinic bond saturation catalyst includes a support including at least one of Al₂O₃, SiO₂, Cr₂O₃, MgO, TiO₂, activated carbon, carbon fibers, and carbon nanotubes.

Embodiment 9 provides the process according to any one of Embodiments 1-8, wherein the decarboxylation includes the olefinic bond saturation product and at least one solvent or a mixture of solvents contacting a heterogeneous decarboxylation catalyst.

Embodiment 10 provides the process according to Embodiment 9, wherein the catalyst is selected from supported catalysts comprising at least one of a Group VIII metal and a Group VIA metal.

Embodiment 11 provides the process according to Embodiment 10, wherein the catalyst comprises a catalyst at a temperature of about 100°C to about 450°C.

Embodiment 12 provides the process according to Embodiment 11, wherein the catalyst comprises a catalyst at a pressure of about atmospheric pressure to about 150 MPa.

Embodiment 13 provides the process according to Embodiment 12, wherein in the catalyst comprises a catalyst in an atmosphere of at least one of an inert gas or an inert gas-hydrogen mixture.

Embodiment 14 provides the process according to any one of Embodiments 9-13, wherein in the decarboxylation process includes an inert gas-hydrogen mixture ranging in hydrogen concentration of about 1 % to about 15% hydrogen.

Embodiment 15 provides the process according to any one of Embodiments 9-14, wherein the catalyst used for the decarboxylation process comprises at least one of Pd, Ni, NiMo, or CoMo.

Embodiment 16 provides the process according to any one of Embodiments 9-15, wherein the catalyst used for the decarboxylation process comprises at least one of Al₂O₃, SiO₂, Cr₂O₃, MgO, TiO₂, activated carbon, carbon fibers, and carbon nanotubes.

Embodiment 17 provides the process according to any one of Embodiments 9-16, wherein the solvent in the decarboxylation process comprises at least one selected from a group consisting of paraffin(s), isoparaffin(s), naphthene(s), aromatic(s), and the recycled product of the decarboxylation reaction process.

Embodiment 18 provides the process according to any one of Embodiments 9-17, wherein the solvent used in the decarboxylation process comprises at least one product of the recycled decarboxylation reaction process.

Embodiment 19 provides the process according to any one of Embodiments 1-18, wherein hydrogen from a reactor vessel of the olefinic bond saturation process includes at least one of hydrogen recovered from the process, hydrogen recycled from the process, and hydrogen returned to an inlet of the reactor vessel of the olefinic bond saturation process.

Embodiment 20 provides the process according to any one of Embodiments 1-19, wherein a ratio of moles of the paraffin product generated by decarboxylation reactions and decarbonylation reactions to moles of the paraffin product generated by reduction reactions and deoxygenation reactions is about 0.3:1 - 3.2:1.

Embodiment 21 provides the process according to any one of Embodiments 1-20, wherein a weight percent conversion of at least one of the unsaturated fatty acids and the unsaturated fatty acid esters to the paraffin product is about 20 wt% to about 100 wt%.

Embodiment 22. The process according to any one of Embodiments 1-21, wherein the olefin bond saturation process and the deoxygenation process together consume about 0.5 g to about 2.5 g of hydrogen per 100 grams of paraffin product produced.

Embodiment 23 provides the method of any one or any combination of Embodiments 1-22 optionally configured such that all elements or options recited are available to use or select from.

## Claims

1. A process for the manufacture of saturated hydrocarbons, the process comprising:
performing a olefinic bond saturation process on a feedstock comprising at least one of unsaturated fatty acids and unsaturated fatty acid esters, optionally comprising at least one of saturated fatty acids, saturated fatty acid esters, and triacylglycerides; and
performing a deoxygenation process on the feedstock including a decarboxylation process to yield a mixture of paraffins.

2. The process according to claim 1, wherein the feedstock comprises at least about 20% by weight of unsaturated fatty acids or fatty acid alkyl esters.

3. The process according to any one of claims 1-2, wherein the feedstock comprises about 50% to about 100% by weight of unsaturated fatty acids or fatty acid alkyl esters.

4. The process according to any one of claims 1-3, wherein the fatty acids or fatty acid alkyl esters used as the feedstock have carbon numbers ranging from 8 to 26.

5. The process according to any one of claims 1-4, wherein the olefinic bond saturation is carried out in the presence of a supported hydrogenation catalyst comprising one or more Group VIII metals of the periodic table and Group VIA metals of the periodic table, at a temperature of about 50°C to about 250°C at a pressure using hydrogen at a pressure of about 0.1 MPato about 30 MPa.

6. The process according to claim 5, wherein the catalyst for olefinic bond saturation comprises at least one of Ni, Mo, Pd, and Co.

7. The process according to any one of claims 1-6, wherein the decarboxylation includes the olefinic bond saturation product and at least one solvent or a mixture of solvents contacting a heterogeneous decarboxylation catalyst.

8. The process according to claim 7, wherein the catalyst is selected from supported catalysts comprising at least one of a Group VIII metal and a Group VIA metal.

9. The process according to claim 8, wherein the catalyst comprises a catalyst at a temperature of about 100°C to about 450°C.

10. The process according to claim 9, wherein the catalyst comprises a catalyst at a pressure of about atmospheric pressure to about 150 MPa.

11. The process according to claim 10, wherein in the catalyst comprises a catalyst in an atmosphere of at least one of an inert gas or an inert gas-hydrogen mixture.

12. The process according to any one of claims 7-11, wherein the catalyst used for the decarboxylation process comprises at least one of Pd, Ni, NiMo, CoMo, Al₂O₃, SiO₂, Cr₂O₃, MgO, TiO₂, activated carbon, carbon fibers, and carbon nanotubes.

13. The process according to any one of claims 1-12, wherein a ratio of moles of the paraffin product generated by decarboxylation reactions and decarbonylation reactions to moles of the paraffin product generated by reduction reactions and deoxygenation reactions is about 0.3:1 - 3.2:1.

14. The process according to any one of claims 1-13, wherein a weight percent conversion of at least one of the unsaturated fatty acids and the unsaturated fatty acid esters to the paraffin product is about 20 wt% to about 100 wt%.

15. The process according to any one of claims 1-14, wherein the olefin bond saturation process and the deoxygenation process together consume about 0.5 g to about 2.5 g of hydrogen per 100 grams of paraffin product produced.
